# EUROPEAN PATENT APPLICATION

(11) **EP 0 742 012 A2**
(43) Date of publication of application: **13.11.1996**
(21) Application number: 96107224.6
(22) Date of filing: 08.05.1996
(51) Int. Cl.: A61K 35/78, A61K 31/35, A61K 31/70, A61K 35/84, A61K 38/00, A61K 31/355, A61K 31/19

(54) **Pharmaceutical composition containing substance inhibiting HSP47 production**

(30) Priority: 10.05.1995 JP 136027/95; 10.05.1995 JP 136028/95; 10.05.1995 JP 136029/95; 29.06.1995 JP 186302/95; 27.07.1995 JP 210935/95; 28.07.1995 JP 211274/95
(71) Applicant: Kureha Chemical Industry Co., Ltd., Tokyo 103 (JP)
(72) Inventor: Kiyosuke, Yoichi, Tokyo 169 (JP); Shirakami, Toshiharu, Kodaira-shi, Tokyo 187 (JP); Morino, Masayoshi, Tokyo 179 (JP); Yoshikumi, Chikao, Kunitachi-shi, Tokyo 186 (JP)
(74) Representative: Cohausz & Florack

(57) **Abstract**

A pharmaceutical composition comprising a substance inhibiting HSP47 production, selected from the group consisting of a malt extract, a flavonoid compound, a protein-bound-polysaccharide obtained from a fungus belonging to Coriolus versicolor, a paeoniflorin derivative, a tocopherol derivative, and a ferulic acid derivative, and a pharmaceutically acceptable carrier is disclosed. The substance inhibiting HSP47 production can efficiently improve physiological conditions of a patient suffering from diseases exhibiting pathosis of overproduction of the extracellular matrix, and efficiently treat such diseases. Further, the substance is useful for preventing or treating various diseases accompanied with abnormal growth of the vascularization.

## Description

### BACKGROUND OF THE INVENTION

### 1. Field of the Invention

The present invention relates to a pharmaceutical composition containing a substance inhibiting production of a heat shock protein having a molecular weight of 47 KDa (hereinafter referred to as HSP47).

The substance inhibiting HSP47 production can inhibit particularly collagen synthesis in organs to efficiently improve physiological conditions of patients suffering from diseases exhibiting pathosis of overproduction of an extracellular matrix, for example, liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis, or to efficiently treat said diseases.

### 2. Description of the Related Art

Recently, diseases exhibiting pathosis of overproduction of an extracellular matrix, such as collagen, have become a considerable problem. The diseases exhibiting pathosis of overproduction of an extracellular matrix include, for example, liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis.

It is said that the number of deaths by liver cirrhosis rises to about twenty thousands a year in Japan. The liver cirrhosis is a generic name of the diseases wherein a liver is stiffen by a connective tissue growth. The liver cirrhosis is a diffuse hepatic fibrosis throughout the liver, and is said to be the ultimate in the chronic liver disease. More particularly, the chronic hepatitis prolongedly suffering from liver disorders, such as inflammation, is accompanied by considerable overproduction of an extracellular matrix, particularly the type I collagen, in the fibroblasts and/or Ito cells or the like, and the liver is led to fibrosis. If the fibrosis of the liver chronically progresses, normal regeneration of the liver becomes progressively inhibited. The fibroblasts and the extracellular matrices mainly composed of the type I collagen are substituted for the hepatocyte, and predominantly occupy the liver tissue. Then, the liver cirrhosis comprising a lot of pseudolobules is caused. When the liver cirrhosis progresses, fibrous septums spread the whole liver and cause the hemostasis. The hemostasis partially causes further degeneration of the liver parenchymal cells. As above, a vicious cycle of the liver cirrhosis continues, and a lot of collagen fibers are formed in the liver by many causes, such as alcohol, virus, autoimmunity, or the like. Then, the necrosis and dysfunction of the hepatocyte are caused, and the patient of the liver cirrhosis ultimately dies. The type I collagen accounts for about 2 % in the whole proteins in a normal liver, but for 10 to 30 % in the liver cirrhosis.

The interstitial lung disease is a syndrome characterized by a chronic inflammation in a lower respiratory tract including not only alveoli and alveolar ducts, but also respiratory bronchioles and terminal bronchioles, such as alveolitis, and the fibrosis of the stroma and the fibrosis in the alveoli as the result therefrom. The term 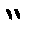 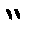 interstitial lung disease*"* includes, for example, interstitial pneumonia, diffuse interstitial lung disease, such as pulmonary fibrosis, idiopathic pulmonary fibrosis, permeability pulmonary edema, interstitial pneumonia associated with collagen vascular disease, sarcoidosis, or the like. In the interstitial lung disease, overproduction and accumulation of the extracellular matrices are observed in the fibrotic tissues. In the fibrotic tissues of the interstitial lung disease, the type I and type III collagens are remarkably accumulated in hypertrophic stroma. Particularly, the type III collagen is accumulated in the hypertrophic alveolar septa at an early stage of the fibrosis. The type I collagen is increased at a late stage and becomes a major collagen. The basement membrane is destroyed at the early stage, and the invasion of the collagen fibers into the alveolar space is observed.

The chronic renal failure means the conditions that as a result of the chronic nephritic syndrome, renal function is damaged too severe to maintain endogenous homeostasis. In a pathological point of view, the progress of the chronic renal failure is the progress of the glomerular sclerosis and the fibrosis of the stroma. In the glomerulosclerosis, the hyperplasia of the extracellular matrix is caused mainly in the mesangial region. In the mesangial sclerosis, the components in the glomerular basement membrane, such as the type IV collagen, is remarkably increased in comparison with a normal mesangial region, and the hyperplasia of the type I collagen which is the component of the stroma is observed in the regions corresponding to those of the scleroma. The chronically progressing glomerular sclerosis is mainly caused by the hyperplasia of the extracellular matrix. The disease leading to the chronic renal failure includes, for example, IgA nephropathy, focal glomerulosclerosis, membranoproliferative nephritis, diabetic nephropathy, chronic interstitial nephritis, chronic glomerulonephritis, or the like.

Regarding the other diseases exhibiting pathosis of overproduction of an extracellular matrix, such as the postoperative scar, burn scar, keloid or hypertrophic scar remaining after a traffic accident, scleroderma, or arteriosclerosis, it is believed that abnormal overproduction of collagen by some cause aggravates the fibrosis and then the induration of the tissue to cause the diseases.

It was reported that the basement membrane and the collagen synthesis therein play an important role in the vascularization [Maragoudakis, E., Sarmonika, M., and Panoutsacopoulous, M., "J. Pharmacol. Exp. Ther.", 244: 729, 1988; Ingber, D. E., Madri, J. A., and Folkman, J., "Endocrinology", 119: 1768, 1986]. The examples known as the diseases caused by the vascularization are, for example, diabetic retinopathy, retrolental fibroplasia, vascularization due to corneal transplantation, glaucoma, eye tumor, trachoma, psoriasis, pyogenic granuloma, hemangioma, angiofibroma, hypertrophic scar, granulation, rheumatoid arthritis, scleredema, atherosclerosis, or other tumors.

Although the diseases exhibiting pathosis of overproduction of the extracellular matrix, such as collagen, have become a considerable problem as above, an agent for inhibiting the production of the extracellular matrix, such as an agent for inhibiting the production of collagen, which is satisfactory for side effects, pharmacological effect, and so on, has not been developed.

A heat shock protein (HSP) or a stress protein is produced in a cell, when some stress, such as heat, a chemical substance, or radiation is added to the cell. HSP includes many types, and may be generally classified into four families in view of the molecular weights, namely, an HSP90 family, an HSP70 family, an HSP60 family, and a small HSP family. Some of these proteins are synthesized constitutirely, and their roles under normal conditions have shown to be essential for physiological functions including folding and unfolding of proteins, assembly of the subunits of proteins, and membrane transport of proteins. These functions of the heat shock protein have led to their designation as molecular chaperone.

HSP47 which was found by Nagata, et al. in 1986 is a basic protein (pI = 9.0) having a molecular weight of 47 kDa. Many reports show that when the production of the HSP47 is increased, the production of collagen is increased ["J. Biol. Chem.", 261: 7531, 1986; "Eur. J. Biochem.", 206: 323, 1992; "J. Biol. Chem.", 265: 992, 1990; "J. Clin. Invest.", 94: 2481, 1994]. More particularly, it is believed that HSP47 serves as a molecular chaperone specific for collagen which is presumably involved in the processing and/or the triple helix formation of procllagen in the endoplasmic reticulum, or transporting or secreting of procollagen from an endoplasmic reticulum to a Golgi apparatus. Thus, the increased production of HSP47 stimulates accumulation of collagen molecules in the extracellular matrix. As above, the collagen-bound heat shock protein, HSP47, is closely associated with collagen which is an extracellular matrix protein, in view of production and function.

### SUMMARY OF THE INVENTION

The inventors of the present invention engaged in intensive studies to provide an agent for inhibiting the production of the extracellular matrix which can efficiently improve physiological conditions of patients suffering from the diseases exhibiting pathosis of overproduction of an extracellular matrix, for example, liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis, and efficiently treat said diseases.

As explained above, a main lesion of the fibrosis, such as liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis is understood to be pathosis that the extracellular matrix is remarkably increased in an organ. It is believed that fibrosis accompanied with the diseases exhibiting pathosis of overproduction of the extracellular matrix, such as liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis, is caused by the increased biosynthesis of collagen or the reduced degradation of collagen. For example, synthesis of the types I, III and IV collagens is activated in the fibrosis of the liver, and particularly, the activation of the synthesis of the type I collagen which is a major component is important.

Under the circumstances, the present inventors unexpectedly found the particular substances which can specifically inhibit the production of HSP47 in cells belonging to the tissue exhibiting pathosis. More particularly, the present inventors found that the production of HSP47 in a cell, and the production of collagen in an organ can be inhibited by administering each of the particular substances, whereby the diseases exhibiting pathosis of overproduction of the extracellular matrix, such as liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis can be treated. The present invention is based on the findings.

Accordingly, the object of the present invention is to provide an agent for inhibiting the production of HSP47 which is the collagen-specific molecular chaperone playing important roles in the maturing and transporting processes of collagen in a cell, which agent can efficiently treat the diseases exhibiting pathosis of overproduction of the extracellular matrix, such as liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatiod arthritis.

Other objects and advantages will be apparent from the following description.

The present invention relates to a pharmaceutical composition comprising a substance inhibiting HSP47 production, selected from the group consisting of a malt extract, a flavonoid compound, a protein-bound-polysaccharide obtained from a fungus belonging to Coriolus versicolor, a paeoniflorin derivative, a tocopherol derivative, and a ferulic acid derivative, and a pharmaceutically acceptable carrier.

Further, the present invention relates to a method for preventing or treating a disease exhibiting pathosis of overproduction of an extracellular matrix, comprising administering to a mammal in need thereof said substance inhibiting HSP47 production, in an effective amount for prevention or treatment thereof.

Still further, the present invention relates to use of said substance inhibiting HSP47 production, for preparing a pharmaceutical composition.

### BRIEF DESCRIPTION OF DRAWINGS

Figure 1 is a chromatogram of the dried malt extract prepared in Example 2.

Figure 2 is an infrared absorption spectrum of the dried malt extract prepared in Example 2.

Figure 3 is a ¹H-nuclear magnetic resonance spectrum of the dried malt extract prepared in Example 2.

Figure 4 is an ultraviolet-visible absorption spectrum of the dried malt extract prepared in Example 2.

### DESCRIPTION OF THE PREFERRED EMBODIMENTS

The present invention will be explained in detail hereinafter.

### (1) Malt extract

The malt extract which may be used as the substance inhibiting production of HSP47 in the present invention is not particularly limited. It is possible to use, for example, a commercially available malt extract without further treatment, or after removing low molecular weight fractions by dialysis or the like.

It is preferable to use a malt extract prepared by saccharifying a malt, filtering the saccharified malt, extracting the filter cake with water, a hydrophilic solvent or a mixture thereof, and removing low molecular weight fractions from the liquid extract. It is more preferable to use a malt extract prepared by performing the above procedure except that the extraction of the filter cake from the saccharified malt with water, a hydrophilic solvent or a mixture thereof is carried out in an alkaline condition, the resulting liquid extract is neutralized, the resulting precipitated substances are removed, and low molecular weight fractions are removed from the resulting supernatant. It is still more preferable to use a malt extract prepared by performing the above procedure except that after extracting the filter cake with water, the hydrophilic solvent or the mixture thereof, the resulting liquid extract is adjusted to pH 3 to 5, the precipitated proteins are removed, the resulting supernatant is neutralized, the resulting precipitated substances are removed, and low molecular weight fractions are removed from the resulting supernatant. It is most preferable to use a malt extract prepared by performing the above procedure except that the extraction of the filter cake from the saccharified malt with water, a hydrophilic solvent or a mixture thereof is carried out in an alkaline condition, the resulting liquid extract is acidified to pH 3 to 5, the precipitated proteins are removed, the resulting supernatant is neutralized, the resulting precipitated substances are removed, and low molecular weight fractions are removed from the resulting supernatant.

It is particularly preferable to use a physiologically active substance derived from a malt extract which is prepared by saccharifying a malt, filtering the saccharified malt, extracting the filter cake with water, a hydrophilic solvent or a mixture thereof, optionally carrying out the above neutralizing step, or the above acidifying and subsequent neutralizing steps, and removing low molecular weight fractions from the resulting liquid extract, and which has
(a) the molecular weight of about 10000 or more, (b) infrared absorption spectrum having peaks at 3600 - 3200 cm⁻¹ and 1700 - 1600 cm⁻¹, (c) proton nuclear magnetic resonance spectrum having peaks at 0.5 - 6.0 ppm and 6.0 - 8.5 ppm, and (d) ultraviolet absorption spectrum having peaks at 200 - 250 nm and 250 - 350 nm, and which shows (e) a positive indication in a phenol-sulfuric acid color reaction, and (f) a positive indication in a copper-Folin color reaction.

In the present invention, a malt prepared by germinating the grains including gramineous crops may be used. These grains include, for example, barley, wheat, rye, oats, naked barley or corn. The saccharification may be carried out, using the malt as it is, or after pulverizing, or if necessary, after adding starch or warm water. The resulting saccharified malt is filtered. The resulting filtrate of the saccharified malt is generally called wort and used as a starting material for beer. The other filter cake of the saccharified malt is generally used as a livestock feed, and may be used as the starting material of the malt extract in the present invention.

Then, the resulting filter cake of the saccharified malt may be extracted with water, an alkaline aqueous solution, a hydrophilic solvent, a mixture of water and a hydrophilic solvent, or a mixture of an alkaline aqueous solution and a hydrophilic solvent at ordinary temperature or elevated temperature to obtain the extract. The hydrophilic solvent for extraction may be, for example, a lower alcohol, such as methyl alcohol or ethyl alcohol, or a ketone, such as acetone. For example, the extraction is preferably carried out for several hours or overnight, by adding 10 to 1000 parts by weight of water, a warmed alkaline aqueous solution, or a hydrophilic solvent to 100 parts by weight of dried or wet filter cake of the saccharified malt. The extraction step may be carried out with stirring or ultrasonic treatment, or with adding a surface active agent, to enhance the extracting effect.

It is preferable to perform the extraction under alkaline condition, preferably at pH 10 to 14, neutralize the resulting liquid extract to, for example, pH 6.5 to 7.5, and remove the precipitates. For example, the extraction with 0.45 to 0.55 N sodium hydroxide aqueous solution can enhance the extracting efficiency, and improve water-solubility of the resulting extract without affecting the quality of the filter cake of the saccharified malt.

After extraction with water, the hydrophilic solvent or the mixture thereof, it is possible to acidify the resulting liquid extract with an inorganic acid, such as hydrochloric acid, or an organic acid, such as oxalic acid to, for example, pH 3 to 5, remove precipitated proteins, neutralize the resulting supernatant as above and further remove precipitates.

Further, it is possible to carry out the extraction under alkaline condition, preferably at pH 10 to 14, acidify the resulting liquid extract with an inorganic acid, such as hydrochloric acid, or an organic acid, such as oxalic acid to, for example, pH 3 to 5, remove precipitated proteins, neutralize the resulting supernatant as above, and further remove precipitates.

The extract prepared by the above extraction procedure, or optionally, the above neutralizing step, or the above acidifying and subsequent neutralizing steps, may be used as the substance inhibiting HSP47 production in the present invention. However, it is preferable to use the extract after removing the low molecular weight fractions from the above extract by, for example, dialysis, salting-out, ultrafiltration, reverse osmosis, gel filtration, precipitation with an organic solvent, or the like. The fractions having molecular weights of not more than 10,000 are preferably removed.

The malt extract may be used without further treatment or after concentrated. Further, lyophilized product or spray-dried powder thereof may also be used.

An acute toxicity test for mice by oral administration showed that the malt extract which may be used as the substance inhibiting HSP47 production in the present invention does not have toxicity.

### (2) A flavonoid compound

The flavonoid compound which may be used as the substance inhibiting HSP47 production in the present invention is not particularly limited, and a known flavonoid compound may be used. The flavonoids which may be used in the present invention are, for example, chalcones, flavanones, flavones, flavonols, flavanonols, flavanols (catechins), isoflavones, or anthocyans, or the like. The flavonoid compound may be used alone or in combination with one or more various flavonoid compounds.

As the chalcones, there may be mentioned, for example, isookanin, isocarthamin, isosalipurpin, isobutrin, isoliquiritin, okanin, chalcone, carthamin, coreopsin, stillopsidin, neosakuranin, butein, pedicin, pedicellin, marein, lanceolin, or lanceoletin, or the like.

As the flavanones, there may be mentioned, for example, alpinetin, isocarthamidin, isosakuranin, isosakuranetin, isopedicin, eriodictyol, carthamidin, cryptostrobin, sakuranin, sakuranetin, salipurpin, dihydrowogonin, cyrtominetin, strobopinin, naringin, naringenin, neocarthamin, neohesperidin, pinostrobin, pinocembrin, farrerol, butin, butrin, flavanookanin, flavanomarein, flavanolanceoletin, flavanone, prunin, hesperidin, hesperetin, verecundin, homoeriodictyol, poncirin, matteucinol, liquiritigenin, or liquiritin, or the like.

As the flavones, there may be mentioned, for example, acaciin, acacetin, apiin, apigenin, wogonin, oroxylin-A, galuteolin, chrysin, chrysoeriol, glucoluteolin, genkwanin, cosmosiin, diosmin, diosmetin, scutellarin, scutellarein, strobochrysin, tectochrysin, tricin, toringin, nobiletin, baicalin, baicalein, flavone, primetin, pectolinarigenin, pectolinarin, pedaliin, pedalitin, ponkanetin, linarin, luteolin, rhoifolin, lotusin, or lotoflavin, or the like.

As the flavonols, there may be mentioned, for example, azaleatin, azalein, astragalin, avicularin, afzelin, ayanin, icariin, icaritin, izalpinin, isoquercitrin, isorhamnetin, erianthin, auranetin, kanugin, galangin, karanjin, gardenin, cannabiscitrin, xanthorhamnin, chrysosplenetin, quercituron, quercitrin, quercimeritrin, quercetagitrin, quercetagetin, quercetin, keyakinin, kaempferid, kaempferitrin, kaempferol, gossypitrin, gossypin, gossypetin, spiraeoside, datiscetin, thapsin, tangeritin, tambulin, tambuletin, ternatin, trifolin, narcissin, noricariin, noricaritin, patuletin, hibiscitrin, hibiscetin, hyperin, fisetin, flavonol, persicarin, herbacitrin, herbacetin, miquelianin, myricitrin, myricetin, meratin, melisimplin, melisimplexin, meliternatin, meliternin, morin, rhamnazin, rhamnetin, rhamnocitrin, rutin, reynoutrin, robinin, or robinetin, or the like.

As the flavanonols, there may be mentioned, for example, astilbin, alpinon, aromadendrin, ampeloptin, isoengelitin, engelitin, keyakinol, dihydrorobinetin, strobobanksin, taxifolin, pinobanksin, phellamurin, phellamuretin, or fustin, or the like.

As the flavanols (catechins), there may be mentioned, for example, afzelechin, epiafzelechin, epicatechin, epicatechin gallate, epigallocatechin, epigallocatechin gallate, catechin, catechin gallate, gallocatechin, or gallocatechin gallate, or the like.

As the isoflavones, there may be mentioned, for example, isoflavone, irigenin, iridin, osajin, ononin, genistin, genistein, santal, sophorabioside, sophoricoside, daidzin, daidzein, tectorigenin, tectoridin, biochanin A, pseudobaptigenin, pseudobaptisin, prunusetin, prunetin, pomiferin, or formononetin, or the like.

As the anthocyans, there may be mentioned, for example, awobanin, idaein, ilicicyanin, oenin, chrysanthemin, gesnerin, gesneridin, keracyanin, salvianin, cyanidin, cyanin, delphinidin, delphinin, delphin, negretein, violanin, hirsutidin, hirsutin, primulin, prunicyanin, paeonidin, paeonin, petunidin, petunin, pelargonidin, pelargonin, malvidin, or malvin, or the like.

The preferred flavonoids which may be used as the substance inhibiting HSP47 production according to the present invention are quercetin [i.e., 2-(3,4-dihydroxyphenyl)-3,5,7-trihydroxy-4H-1-benzopyran-4-one], rutin (i.e., quercetin-3-rutinoside), baicalein (i.e., 5,6,7-trihydroxy-2-phenyl-4H-1-benzopyran-4-one), or catechins. The preferred catechins which may be used as the substance inhibiting HSP47 production according to the present invention are (+) catechin, (+) gallocatechin, (+) catechin gallate, (+) gallocatechin gallate, (-) epicatechin, (-) epigallocatechin, (-) epicatechin gallate, (-) epigallocatechin gallate. As the flavonoid which may be used as the substance inhibiting HSP47 production according to the present invention, a pure stereoisomer of flavonoid or a mixture of the stereoisomers thereof may be used.

The flavonoids which may be used as the substance inhibiting HSP47 production according to the present invention may be prepared by a chemical synthesis or extraction from a naturally-occurring material followed by purification. Alternatively, commercially available flavonoids may be used. The catechins which may be used as the substance inhibiting HSP47 production according to the present invention are particularly known as tea cathchins. The catechin prepared by extraction from a naturally-occurring material and purification is, not limited to, but preferably the catechin extracted for tea.

Because the tea plant contains the tea catechins, the tea extract may be used as the substance inhibiting HSP47 production according to the present invention.

The term 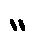 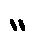 tea*"* used herein means a raw or dried whole plant or a part (for example, a leaf, wood, root, or fruit) of Cammellia sinensis, (L) O. Kuntze, or a partially or completely fermented product thereof, which may be used alone or in combination thereof. Various types of the tea leaves may be used for extraction. For example, products obtained at any stages of an ordinary tea manufacturing process, i.e., those from a raw tea leaf to a final tea product (dried tea) may be used. Further, tea products at any fermentation stages, for example, a fermented tea such as black tea, a semi-fermented tea such as an oolong tea, or a non-fermented tea such as green tea may be used.

The tea extract which contains the tea catechins may be used as the substance inhibiting HSP47 production in the present invention, and therefore, the tea crude extract may be used. The tea crude extract may be obtained by extracting tea with warmed water, preferably hot water, or an organic solvent. As the organic solvent, there may be mentioned, for example, a lower alcohol, such as methyl alcohol, ethyl alcohol, n-propyl alcohol, isopropyl alcohol or butyl alcohol, a lower ester, such as methyl acetate, ethyl acetate, propyl acetate or butyl acetate, or a ketone, such as acetone or methyl isobutyl ketone. The organic solvents can be used alone or in combination thereof. Further, the anhydrous or aqueous organic solvent may be used.

As the extraction with water or the organic solvent, an ordinary extraction method for obtaining crude drugs may be used. For example, it is preferable to add 5 to 20 parts by weight of water or organic solvent to 1 part by weight of dried tea leaf, and heat the whole under reflux at the temperature below the boiling point with stirring. The extraction procedure may be carried out generally for 5 minutes to 7 days, preferably for 10 minutes to 24 hours. If necessary, an auxiliary means, such as stirring, may be used to shorten the extraction time.

The resulting extract with water or the organic solvent may be treated by a suitable process, such as filtration or centrifugation, to remove insoluble materials. Products obtained by treating the above extract further with various organic solvents or adsorbents may also be used as the tea extract of the substance inhibiting HSP47 production in the present invention. The tea extract may be concentrated, pulverized by drying, or purified by being crystallized from cold water.

The resulting tea extract contains a mixture of catechins in tea (particularly, tea leaf), i.e., tea catechins, such as catechin, epicatechin, gallocatechin, epigallocatechin, catechin gallate, epicatechin gallate, epigallocatechin gallate, gallocatechin gallate, as well as impurities from the starting tea material used.

Among the flavonoid compounds which may be used as the substance inhibiting HSP47 production in the present invention, quercetin has an acute toxicity (LD₅₀) of 160 mg/kg for a mouse in an oral administration (the Merck Index, 11th Ed.; Merck, P. 1278), and rutin has an acute toxicity (LD₅₀) of 950 mg/kg for a mouse in an intravenous injection (the Merck Index, 11th Ed.; Merck, P. 1319). Further, toxicity was not observed in the tea catechins which may be used as the substance inhibiting HSP47 production in the present invention.

### (3) Protein-bound-polysaccharide

The protein-bound-polysaccharide which may be used as the substance inhibiting HSP47 production in the present invention is obtained from a fungus belonging to Coriolus versicolor in Basidiomycetes. The protein-bound-polysaccharide from the fungus belonging to Coriolus versicolor which may be used as the substance inhibiting HSP47 production in the present invention is disclosed in, for example, Japanese Examined Patent Publications (Kokoku) No. 46-17149, No. 51-36322, No. 56-14274, No. 56-14275, and No. 56-14276.

Thus, the protein-bound-polysaccharide is obtained by extracting mycelia, broth, or fruit bodies of the natural or cultivated fungus belonging to Coriolus versicolor, with an aqueous solvent. The aqueous solvent is an extracting solvent mainly containing water, and thus includes water, or a solution containing one or more water-soluble acids, bases, salts, or organic solvents.

The cultivation procedures may comprise transferring a part of a rotten plant to which the fungi adhered, a tissue of the fruit bodies sprouted from the rotten plant, or a spore thereof to a suitable agar medium, cultivating for several weeks, and repeating the above transferring and cultivating steps twice or three times to confirm no contamination with other microorganisms. The resulting fungi are used as a seed to inoculate a liquid or solid medium, and the cultivation is carried out. The cultivation in the liquid medium includes, for example, a standing, shaking, bubbling, or bubbling/stirring cultivation. The solid medium is, for example, agar, gelatin, starch, sawdust, wood, pulp, sponge, synthetic resin, rubber, or sand, which may be used in combination thereof. The liquid or solid medium may be used for cultivation of the fungus, but the liquid medium is very preferable in view of workability and productivity. The medium containing components used for ordinary cultivation, or containing various nutritives necessary to grow the fungus may be used in the present invention. As the carbon source, for example, glucose, maltose, lactose, sucrose, starch, or molasses may be used. As the nitrogen source, for example, a nitrogen-containing inorganic or organic substance, such as peptone, meat extract, yeast extract, yeast, corn steep liquor, ammonium salts, or urea may be used. Examples of inorganic salts are phosphate, magnesium salt, iron salt and so on. The medium can optionally contain vitamins necessary for growth. The initial pH is preferably about 2 to 7. The cultivation is preferably carried out at 20 to 33 °C for 2 to 20 days. It is preferable to carry out the bubbling/stirring cultivation in air bubbling rate of 0.1 to 2.0 liter/liter of medium/min at a stirring rate of 30 to 800 rpm.

The fungus belonging to Coriolus versicolor may be extracted as it is, or generally after pretreated, for example, washed with distilled water, physiological saline, various buffers or the like, dried, degreasing with a lipophilic organic solvent, such as n-hexane, benzene, petroleum ether, chloroform or carbon tetrachloride, and then optionally grinding.

Alternatively, the fungus belonging to Coriolus versicolor may be cultivated by a deep culture in an aqueous liquid medium to obtain the culture mixture of the fungi and the culture products, i.e., a broth. The broth may be dried and extracted with the aqueous solvent, and the liquid extract can be purified by removing the substances having molecular weights of 5000 or less. As above, the desired protein-bound-polysaccharide can be obtained by drying the cultured product, and extracting with the aqueous solvent.

The term 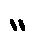 deep culture*"* means a method wherein a cultivation is carried out in a liquid while bubbling and stirring, and thus, the fungi are no grown on the surface of the liquid medium, but in a deep portion. The bubbling rate is generally 0.1 to 2.0 liter/liter of medium/min, and the stirring rate is generally 30 to 800 rpm. The desired protein-bound-polysaccharide can be sufficiently obtained after cultivating for about 7 days. The drying process is carried out at preferably 60 to 150 °C, more preferably 90 to 130 °C, so that the moisture content becomes about 20 % by weight or less. The drying means used in the drying process is not limited, but may be an ordinary drying means, such as a drum dryer, flash dryer, or HITACHI Sevcon.

The mycelia and/or fruit bodies of the natural or cultivated fungus belonging to Coriolus versicolor, or the dried broth may be extracted with an aqueous solvent with or without stirring.

It is preferable to extract the fungus belonging to Coriolus versicolor (the mycelia or fruit bodies of the fungus belonging to Coriolus versicolor) with 0.01 N to 2 N alkaline aqueous solution, and treat the resulting liquid extract by ultrafiltration and/or reverse osmosis to remove substances having molecular weights of 5000 or less therefrom. The amount of the 0.01 N to 2 N alkaline aqueous solution used is not limited, but is preferable a 5- to 200-fold amount to the fungus (dried weight). The fungus may be sufficiently extracted with the 0.01 N to 2 N alkaline aqueous solution at 50 to 100 °C, preferably 80 to 98 °C, for 20 minutes to 10 hours. The extraction procedure may be carried out once, or repeated several times, for example, twice to 10 times, preferably 3 to 8 times, if necessary. The extraction procedure may be carried out with water or dilute alkaline aqueous solution, and further with alkaline aqueous solutions having gradually increasing concentrations. More particularly, the fungus may be treated by a multiple-stage extraction, namely, extracted initially with water or the aqueous solvent containing a trace amount of an alkali, and then stepwise with gradually concentrated alkaline aqueous solutions, to obtain the desired product. In the multiple-stage extraction, the extraction step using the solutions having the same concentration may be repeated. When the extraction procedures are repeated, the total heating time under said temperature range is preferably 20 hours or less, independently of the number of the steps, so as to avoid decomposition of the active ingredients. The alkali used may be sodium, potassium or calcium hydroxide, or aqueous ammonia, but sodium hydroxide is particularly preferable. The resulting liquid extract may be neutralized with a mineral acid, such as dilute hydrochloric acid, in an ordinary manner, and then purified. The purification may be performed for each liquid extract, or the combined liquid extract.

The purification can be carried out by one or more methods, such as dialysis, ultrafiltration, reverse osmosis, gel filtration, ion exchange resin, salting-out with ammonium sulfate, or precipitation with an organic solvent. The ultrafiltration and/or reverse osmosis may be efficiently applied.

The membrane having a nominal fraction molecular weight of 5000 to 15000, and a recovering rate of 98 to 100 % against the standard substance, cytochrome C (molecular weight = 13000) may be used in the ultrafiltration or reverse osmosis. The procedure conditions to purify the liquid extract of the present invention using the above membrane slightly vary with the form of the apparatus used, the amount of the liquid extract to be treated or the like. In the case of ultrafiltration, however, the pressure is generally 0.5 to 5 kg/cm², preferably 1 to 4 kg/cm², and the temperature is generally 5 to 70 °C, although it varies with the properties of the membrane. In the case of reverse osmosis, the pressure is generally 20 to 30 kg/cm², preferably 20 to 25 kg/cm², and the temperature is generally 5 to 20 °C, although it varies with the properties of the membrane. When the liquid extract is purified, the ultrafiltration or reverse osmosis may be applied alone, or together.

After the substances having the molecular weights of 5000 or less are removed by the purification, the extract is spray-dried or lyophilized to obtain a product.

As described above, the protein-bound-polysaccharide may be obtained by extracting mycelia, broth, or fruit bodies that is obtained by culturing the fungus belonging to Coriolus versicolor. The protein-bound-polysaccharide contains about 18 to 38 % by weight of proteins, and has a molecular weight (ultracentrifugal method) of 5,000 or more, preferably 5,000 to 1,000,000.

A typical example of the protein-bound-polysaccharide from the fungus belonging to Coriolus versicolor which may be used as the substance inhibiting HSP47 production in the present invention is called PSK, which is commercially available as "Krestin" (trade mark) from Sankyo Co., Ltd. PSK has been clinically used and proved to prolong the life of a cancer patient (Nakazato, H., et al., "The Lancet", 343: 1122-1126, 1994). Further, PSK is described in "Saikin no Shinyaku (Recent New Medicines)", Vol. 28, 14-16, 1977; and Vol. 29, 96-101, 1978; and "Iyakuhin Yoran (Handbook of Medicines)", 6th Ed., 1346, May, 1979, published by Yakuji Jiho Publishing Co.

The properties of PSK are as follows:

PSK may be prepared by extracting the mycelia of Coriolus versicolor (Fr.) Quél. CM101 strain [FERM-P2412 (ATCC20547)] with an aqueous solvent, such as hot water or alkaline solution (e.g., a solution of hydroxide of alkali metal, such as sodium hydroxide), purifying and then drying the extract. The sugar portion in the major fractions is β-D-glucan. The glucan has a branched structure containing β1→3, β1→4 and β1→6 bonds, and mainly comprises glucose and mannose. Further, PSK contains about 18 to 38 % by weight of proteins. In the amino acid composition of the proteins, there are many acidic amino acids, such as aspartic and glutamic acids, and many neutral amino acids, such as valine and leucine, but a few basic amino acids, such as lysine and arginine. PSK is soluble in water, but hardly soluble in methanol, pyridine, chloroform, benzene or hexane. PSK begins to be gradually decomposed at about 120 °C.

As a starting material of the protein-bound-polysaccharide, in addition to the above Coriolus versicolor CM101 strain, other fungi strains belonging to Coriolus versicolor (for example, FERM-P Nos. 2413 to 2426), Coriolus consors (Berk.) Imaz., Coriolus pubescens (Fr.) Quél., Coriolus biformis (Kiotz.) Pat., Coriolus hirsutus (Fr.) Quél., Coriolus conchifer (Schw.) Pat., Coriolus pargamenus (Fr.) Pat. and the like may be used.

### (4) A paeoniflorin derivative

Paeoniflorin, a typical example of the paeniflorin derivative which may be used as the substance inhibiting HSP47 production according to the present invention, is a glycoside (C23H28O11=480.47) of the formula: 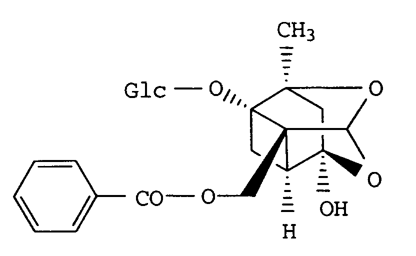 wherein Glc is a glucose residue, and is contained in a crude drug, for example, peony root. Paeoniflorin includes many stereoisomers, and one of the stereoisomers or the mixture thereof may be used as the substance inhibiting HSP47 production in the present invention.

Paeoniflorin which may be used as the substance inhibiting HSP47 production in the present invention may be prepared by chemical synthesis or extraction of a naturally-occurring material followed by purification of the extract. Alternatively, a commercially available paeoniflorin may be used. When the paeoniflorin is extracted from a natural material, the whole plant containing paeoniflorin or a part thereof, such as a whole plant, leaf, root, rhizome, stem, root bark or flower, is extracted without further treatment, or after simple treatment, such as drying, cutting or pulverizing to prepare crude drugs. Extraction conditions are not limited, so long as they are the conditions generally used for plant extraction.

The paeoniflorin extracted from a crude drug as the substance inhibiting HSP47 production is not particularly limited to, but is preferable peony root. The "Paeonia lactiflora Pallas" is a plant, whereas the "peony root" or "peony root; PAEONIAE RADIX" is a crude drug. Thus, the "peony root" is a raw or dried root of the plant "Paeonia lactiflora Pallas" or "Paeoniaceae". A part of the peony root may be used alone or in combination thereof.

A peony-root extract which may be used as the substance inhibiting HSP47 production in the present invention is not limited, so long as it contains the above-mentioned paeoniflorin. Thus, a crude peony-root extract may be used as the substance inhibiting HSP47 production in the present invention. Peony root is extracted with, for example, water, such as warm water, preferably hot water, or an organic solvent to prepare the peony root extract which may be used as the substance inhibiting HSP47 production in the present invention. As the organic solvent, there may be mentioned, for example, methanol, ethanol, n-propanol, isopropanol, butanol, methyl acetate, ethyl acetate, propyl acetate, butyl acetate, acetone, methyl isobutyl ketone, petroleum ether, cyclohexane, carbon tetrachloride, toluene, benzene, dichloromethane, chloroform, ether, pyridine, polyethylene glycol, propylene glycol, butylene glycol, or acetonitrile. The organic solvents can be used alone or as a mixture of a suitable combination at a certain proportion. Further, anhydrous or aqueous organic solvents may be used. Preferably, ethanol, methanol, butylene glycol, water-containing alcohol, or acetonitrile/water mixture is used.

As the extraction with water or the organic solvent, an ordinary extraction method for obtaining crude drugs may be used. For example, it is preferable to add 5 to 300 parts by weight of water or organic solvent to 1 part by weight of dried peony root, and heat the whole under reflux at the temperature below the boiling point with stirring. The extraction procedure may be carried out generally for 5 minutes to 7 days, preferably for 10 minutes to 24 hours. If necessary, an auxiliary means, such as stirring, may be used to shorten the extraction time.

The resulting extract with water or the organic solvent may be treated by a suitable process, such as filtration or centrifugation, to remove impurities and obtain a crude extract. The resulting crude extract may be used, without further treatment, as the extracted and purified paeoniflorin from the natural material of the substance inhibiting HSP47 production according to the present invention. In addition to the extract products with hot water or organic solvent, the extract products obtained by treating the above crude extract further with various organic solvents or adsorbents may also be used as the substance inhibiting HSP47 production in the present invention. The peony root extracts including the crude extract and the various purified extract products may be used in the form of the liquid extract without treatment, a concentrated extract obtained by evaporating the solvents, a pulverized product obtained by evaporating the solvents and drying, a viscous product, or a diluted liquid.

The resulting peony root extracts contain paeoniflorin and impurities from the starting peony root.

The paeoniflorin derivatives include, in addition to said paeoniflorin, paeoniflorin derivatives contained in the crude drug, such as oxypaeoniflorin, benzoylpaeoniflorin, benzoyloxypaeoniflorin, albiflorin, and galloylpaeoniflorin; derivatives modified by chemical treatments in the extraction and/or fractionation; and chemically modified derivatives, such as paeoniflorin tetraacetate, paeoniflorin pentaacetate, desbenzoylpaeoniflorin, paeoniflorin tetraacetate tosylate, paeoniflorin monobenzoate triacetate, and paeoniflorin monobenzoate.

An acute toxicity test for mice by oral administration showed that the paeoniflorin which may be used as the substance inhibiting HSP47 production in the present invention does hardly cause death, and that it does not have toxicity.

### (5) A tocopherol derivative

The tocopherol derivative may be used as the substance inhibiting HSP47 production in the present invention. The term 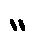 tocopherol*"* used herein includes, for example, α-tocopherol, β-tocopherol, γ-tocopherol, or δ-tocopherol. In the present invention, the tocopherol homologs may be used alone or as a mixture thereof. It is most preferable to use, as the tocopherol, α-tocopherol [3,4-dihydro-2,5,7,8-tetramethyl-2-(4,8,12-trimethyltridecyl)-2H-1-benzopyran-6-ol], namely vitamin E.

The tocopherol derivatives include, for example, tocopherol derivatives contained in a natural material, tocopherol derivatives modified by chemical treatments in the extraction and/or fractionation, and chemically modified tocopherol derivatives. As the tocopherol derivatives contained in a natural material, there may be mentioned, tocotrienol homologs, namely, α-tocotrienol, β-tocotrienol, γ-tocotrienol, or δ-tocotrienol. The tocopherol derivatives modified by chemical treatments in the extraction and/or fractionation, and chemically modified tocopherol derivatives include, for example, tocopherol ester derivatives or tocotrienol ester derivatives.

Examples of the tocopherol ester derivatives are ester compounds of tocopherol with a linear or branched alkyl monoor di-carboxylic acid, preferably a linear or branched alkyl mono- or di-carboxylic acid containing the alkyl moiety having 1 to 30 carbon atoms, a linear or branched alkenyl mono- or di-carboxylic acid, preferably a linear or branched alkenyl mono- or di-carboxylic acid containing the alkenyl moiety having 2 to 30 carbon atoms, an aryl mono- or di-carboxylic acid containing the aryl moiety having 6 to 30 carbon atoms, such as benzoic acid, 5- or 6-membered heterocyclic mono- or di-carboxylic acid containing 1 or 2 nitrogen atoms, such as nicotinic acid, for example, tocopherol acetate, succinate or nicotinate.

The tocopherol derivatives include stereoisomers. In the present invention, any pure stereoisomer or a mixture thereof may be used.

The tocopherol derivatives which may be used as the substance inhibiting HSP47 production in the present invention may be prepared by a chemical synthesis or extraction from a naturally-occurring material followed by purification. Alternatively, commercially available tocopherol derivatives may be used.

### (6) A ferulic acid derivative

Ferulic acid (4-hydroxy-3-methoxycinnamic acid), a typical example of the ferulic acid derivatives which may be used as the substance inhibiting HSP47 production in the present invention, is contained in natural materials, particularly plants, such as rice or adley in the form of the ester. The ferulic acid derivatives include stereoisomers. In the present invention, any pure stereoisomer or a mixture thereof may be used.

The ferulic acid derivatives which may be used as the substance inhibiting HSP47 production in the present invention include, in addition to ferulic acid, for example, ferulic acid derivatives contained in a natural material (particularly plant), ferulic acid derivatives modified by chemical treatments in the extraction and/or fractionation, and chemically modified ferulic acid derivatives. The example is the esters of ferulic acid.

The esters can enhance the solubility in an oily component. Examples of the ferulic acid ester derivatives, particularly pharmaceutically acceptable ferulic acid ester derivatives, are ester compounds of ferulic acid with a linear or branched alkyl or alkenyl alcohol, preferably a linear or branched alkyl or alkenyl alcohol containing the alkyl or alkenyl moiety having 1 to 40 carbon atoms, an aryl alcohol, preferably an aryl alcohol having 6 to 40 carbon atoms, a terpene alcohol, particularly monoterpene alcohol, sesquiterpene alcohol, diterpene alcohol or triterpene alcohol, sterol or trimethyl sterol, for example, ethyl ferulate, 2-ethylhexyl ferulate, allyl ferulate, cetyl ferulate, oleyl ferulate, menthyl ferulate, phenyl ferulate, cholesteryl ferulate, cycloartenol ferulate, 24-methylenecycloartanol ferulate, and so on.

The salt of ferulic acid can enhance the solubility in water. The basic compounds used to form the salt, particularly pharmaceutically acceptable salt, of ferulic acid are, for example, an inorganic salt, such as a hydroxide of alkali or alkaline earth metal, such as lithium, sodium, potassium, magnesium or calcium hydroxide, or ammonium hydroxide, or an organic salt, such as a basic amino acid, such as arginine, lysine or histidine, ornithine, or mono-, di- or ti-ethanol amine. The salt may be added to the pharmaceutical composition of the present invention. Alternatively, the basic compound which can form a salt may be added separately to the pharmaceutical composition of the present invention, to thereby form the salt therein.

The ferulic acid derivatives or pharmaceutically acceptable salts thereof which may be used as the substance inhibiting HSP47 production in the present invention may be prepared by a chemical synthesis or extraction from a naturally-occurring material followed by purification. Alternatively, commercially available derivatives may be used. Ferulic acid can be chemically synthesized by condensing vanillin with malonic acid ( 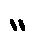 Journal of American Chemical Society*"*, 74:5346, 1952).

In the present invention, the extract from a plant containing the ferulic acid derivatives or pharmaceutically acceptable salts thereof may be used as the substance inhibiting HSP47 production. The plant is not limited, so long as it contains the ferulic acid derivatives or pharmaceutically acceptable salts thereof. The plant includes a pine (Pinus), goldthread (Coptis), asafetida (Ferula), sugarcane (Saccharum), maize (Zea), barley (Hordeum) or rice. It is preferable to use rice. The term 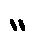 rice*"* used herein means a raw or dried seed of Oryza sativa Linné; Gramineae, which may be used alone or in the form of a mixture of the parts thereof.

The extract from the plant, for example, the extract from the rice with hot water or organic solvent by an ordinary method, may be used as the substance inhibiting HSP47 production in the present invention. The resulting extract, such as the rice extract, may be used as a crude extract without purification. The extract product which is obtained by treating the above crude extract further with various organic solvents or adsorbents and contains the ferulic acid derivatives or pharmaceutically acceptable salts thereof may also be used as the substance inhibiting HSP47 production in the present invention. The resulting extract, such as the rice extract, may be used in the form of the liquid extract without treatment, a concentrated extract obtained by evaporating the solvents, a pulverized product obtained by evaporating the solvents and drying, a purified product by crystallization, a viscous product, or a diluted liquid.

The resulting extracts from the natural material, such as the rice extracts, contain the ferulic acid derivatives or pharmaceutically acceptable salts thereof and impurities from the starting natural material, such as the rice.

When the plant extract, or the ferulic acid derivative or pharmaceutically acceptable salt thereof extracted therefrom is prepared from a natural material, the natural material, for example, the whole plant containing the ferulic acid derivative (particularly the ester compound) or pharmaceutically acceptable salt thereof or a part thereof, such as a whole plant, leaf, root, rhizome, stem, root bark, flower, fruit, or seed, is extracted without further treatment, or after simple treatment, such as drying, cutting or pulverizing. Extraction conditions are not limited, so long as they are the conditions generally used for plant extraction.

The plant extract or the ferulic acid derivative purified therefrom which may be used as the substance inhibiting HSP47 production in the present invention may be prepared, for example, from a natural material as follows:

When the ferulic acid derivative, particularly ferulic acid or ferulic acid ester, is extracted from a natural material and purified, the starting material to be extracted is not limited to, but is preferably, for example, rice, such as a rice bran, a rice germ or a rice seed membrane. For example, the ferulic acid esters can be extracted directly from the rice bran with an alkaline aqueous alcohol. The resulting crude extract may be neutralized with a weak acid to easily precipitate and separate purified ferulic acid esters. The alcohols which may be used are, for example, a lower alcohol, such as methyl alcohol or ethyl alcohol. The alkali is, for example, sodium or potassium hydroxide. The above intermediate extracts containing the ferulic acid esters, for example, the liquid extracts of the rice bran, the rice germ or the rice seed membrane with the alkaline aqueous alcohol may be used as the substance inhibiting HSP47 production in the present invention.

The ferulic acid esters are contained in rice bran oil extracted from the rice bran, rice germ oil extracted from the rice germ, or an oily residue or wax thereof, and thus, such oils may be used as a starting material to be extracted. The ferulic acid esters may be obtained by extracting the oils with a lower alcohol, then extracting the resulting extracted residue with an alkaline lower alcohol solution, and neutralizing the resulting liquid extract with a weak acid to precipitate the ferulic acid esters. The above starting oils containing the ferulic acid esters or the intermediate extract containing the ferulic acid esters may be used as the substance inhibiting HSP47 production in the present invention.

Further, the ferulic acid esters can be obtained by adjusting the above oils containing the ferulic acid esters to pH 12.1 or more under the conditions that saponification is not caused, washing the adjusted oils with non-aqueous organic solvent to remove neutral substances, adjusting the residual liquid to pH 9.0 to 12.0, and then washing the adjusted oils with non-aqueous organic solvent. Examples of the non-aqueous organic solvent are ether, petroleum ether, benzene or other various hydrocarbons.

The crude extract containing the ferulic acid esters can be obtained by extracting the rice bran with a solvent, such as hexane, degumming and dewaxing the resulting oily substance, hydrolyzing the residue with an alkali, neutralizing the hydrolyzed product, separating the solid and liquid parts, distilling the residue, and carrying out an extraction with a solvent, a column treatment and so on. The intermediate products in the above extraction stages which contains the ferulic acid esters and various components from the natural substance, such as the rice, may be used as the substance inhibiting HSP47 production in the present invention. The extract containing the ferulic acid esters obtained by the methods other than those described as above may be used in the present invention.

If necessary, the concentrated ferulic acid esters prepared by the above extracting and purifying procedures may further be purified by the adsorbents or ion exchange resins. In the present invention, γ-oryzanol prepared by thoroughly purifying the above extract, or the component which may be prepared by further purification of γ-oryzanol, such as cycloartenol ferulate or 24-methylenecycloartanol ferulate may be used as the substance inhibiting HSP47 production.

The ferulic acid esters or crude ferulic acid esters obtained as above may be used may be used as the substance inhibiting HSP47 production in the present invention, and may be hydrolized with an alkali to obtain free ferulic acid. The alkali which may be used in the hydrolysis is generally sodium or potassium hydroxide. Further, LiOH, RuOH, Na₂CO₃, K₂CO₃, NaHCO₃ or the like may be used. The ferulic acid esters or crude ferulic acid esters are difficult to be dissolved in water. Thus, it is preferable to use a suitable alcohol as a solvent upon alkaline hydrolysis. Then, a solution containing the alkaline salt of ferulic acid is acidified to precipitate ferulic acid. After filtration, the resulting crude ferulic acid is dissolved in water at an elevated temperature (about 90 to 100 °C). The solution is cooled to precipitate ferulic acid again and obtain free ferulic acid.

Toxicity was not observed in the ferulic acid derivatives which may be used as the substance inhibiting HSP47 production in the present invention.

### (7) Pharmaceutical composition

It is possible to administer one or a mixture of the substances inhibiting HSP47 production according to the present invention, optionally together with preferably a pharmaceutically or veterinarily acceptable ordinary carrier, to an animal, preferably a mammal, particularly human. The formulation is not limited to, but may be, for example, oral medicines, such as powders, fine subtilaes, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method, using, for example, fillers, binders, disintegrating agents, surfactants, lubricants, flowability-enhancers, diluting agents, preservatives, coloring agents, perfumes, tasting agents, stabilizers, humectants, antiseptics, antioxidants, or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate. For example, the capsule may be prepared by mixing and filling 1 part by weight of one or more of the substance inhibiting HSP47 production according to the present invention and 99 parts by weight of lactose. A soft capsule containing vitamin E as an active ingredient in the pharmaceutical composition of the present invention may be prepared by, for example, dissolving vitamin E in plant or synthetic oil, while heating if necessary, and using a coating composition prepared by mixing and melting gelatin, glycerol, and/or antifungal agent in an ordinary method, and a soft-capsule-filling machine. A hard capsule containing vitamin E as an active ingredient in the pharmaceutical composition of the present invention may be prepared by, for example, heating the vitamin E, adsorbing to an fillers such as soft silicic anhydride, adding, if necessary, crystalline cellulose and/or corn starch, admixing the whole, granulating, sieving, and filling the product into a hard capsule by a capsule-filling machine. The mixtures for internal use containing vitamin E as an active ingredient in the pharmaceutical composition of the present invention may be prepared by, for example, emulsifying or solubilizing with a synthetic surfactant such as polyethylene hardened castor oil or a natural surfactant such as lecithin by an ordinary method.

As the parenteral administration, for example, the injection, such as subcutaneous or intravenous injection, or the per rectum administration may be used. The injection is preferable.

When the injections are prepared, for example, water-soluble solvents, such as physiological saline or Ringer's solution, water-insoluble solvents, such as plant oil or fatty acid ester, isotonic agents, such as glucose or sodium chloride, tonicity agents, solubilizing agents, stabilizing agents, antiseptics, suspending agents, emulsifying agents or like may be optionally used, in addition to one or more of the substance inhibiting HSP47 production according to the present invention. For example, 10 mg of one or more of the substance inhibiting HSP47 production according to the present invention and 50 mg of mannitol are dissolved in distilled water to obtain 10 ml of the solution. The solution is sterilized in an ordinary method, distributed into vials at 2 ml per vial, and lyophilized to obtain the injections. The injections may be diluted to prepare injectable liquid, when used.

The pharmaceutical composition may be administered in the form of a sustained release preparation using sustained release polymers. For example, the pharmaceutical composition of the present invention may be incorporated into a pellet made of ethylenevinyl acetate polymers, and the pellet may be implanted into a tissue to be treated.

The amount of the substance inhibiting HSP47 production contained in the pharmaceutical composition of the present invention is not limited. However, the pharmaceutical composition may contain 0.01 to 99 %, preferably 0.1 to 80 % by weight of one or more of the substance inhibiting HSP47 production. The pharmaceutical composition of the present invention containing the tea extract, the paeoniflorin derivative-containing plant extract (particularly, the peony root extract), or the ferulic acid derivative-containing plant extract (particularly, the rice extract) as an active ingredient may be prepared by appropriately adjusting the amount of the flavonoid derivatives, the paeoniflorin derivatives or ferulic acid derivatives, or the pharmaceutically acceptable salts thereof contained therein to the above range. When the pharmaceutical composition of the present invention containing the above extract is prepared as the pharmaceuticals for oral administration, it is preferable to used a pharmaceutically acceptable carrier.

The dose of the pharmaceutical composition of the present invention varies with the kind of the diseases, the age, sex, body weight, symptoms, method of administration, or the like, but one or more of the substance inhibiting HSP47 production according to the present invention may be orally or parenterally administered at a dosage of about 1 mg to 10 g a day for an adult, or the protein-bound-polysaccharide obtained from mycelia, broth or fruit bodies of the fungus belonging to Coriolus versicolor may be orally or parenterally administered at a dosage of about 1 mg to 50 g a day for an adult, while usually divided into one to four dosages.

As explained, one or more of the substances inhibiting HSP47 production contained in the present pharmaceutical composition has a function to specifically inhibit the production of HSP47 in cells. Therefore, when one or more of the substances inhibiting HSP47 production according to the present invention are administered, the biosynthesis of HSP47 in cells is specifically inhibited, and thus, the biosynthesis of collagen is specifically inhibited. As a result, the production of the extracellular matrix is inhibited. Accordingly, one or more of the substances inhibiting HSP47 production according to the present invention may be used for treating or preventing diseases exhibiting pathosis of overproduction of an extracellular matrix accompanied with collagen increase, for example, liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis. In other words, the pharmaceutical composition of the present invention inhibits the production of HSP47 which is a molecular chaperone specific to collagen, and thus collagen.

Further, there was reported that the basement membrane and the collagen synthesis therein play an important role in the vascularization, as above. Therefore, the pharmaceutical composition of the present invention is useful for preventing many diseases caused by abnormal growth in the vascularization, and can be used for the above diseases, for example, diabetic retinopathy, retrolental fibroplasia, vascularization due to cornea] transplantation, glaucoma, eye tumor, trachoma, psoriasis, pyogenic granuloma, hemangioma, angiofibroma, hypertrophic scar, granulation, rheumatoid arthritis, scleredema, atherosclerosis, or other tumors. Furthermore, it was reported that the interstitial stroma having a basic structure of the type I collagen and fibronectin serves, in cancer metastasis, as a guide for the released cancer cells to enter into the vessels in the vicinity [ 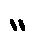 Biotherapy*"*, 7(8): 1181, 1993]. Therefore, the cancer metastasis may be inhibited by administering the pharmaceutical composition of the present invention.

### Examples

The present invention will now be further illustrated by, but is by no means limited to, the following Examples.

### Example 1: Preparation of anti-HSP47 polyclonal antibody

### (1) Preparation of anti-HSP47 polyclonal antibody

A peptide consisting of 15 amino acids corresponding to the amino acid sequence from the second to 16th amino acids in the N-terminus of human HSP47 [hereinafter referred to as human HSP47 peptide (2-16)] was synthesized by an automatic peptide-synthesizer (PSSM-8 System; Shimadzu Corp., Japan). A sensitized antigen was prepared by binding the peptide with lactoglobulin, using succinimidyl 4-(p-maleimidophenyl)butyrate (SMPB) as a crosslinking agent, in an ordinary method ("Biochemistry", 18:690, 1979).

Then, 0.2 ml of phosphate buffered physiological saline [composition: KC1 = 0.2 g/l, KH₂PO₄ = 0.2 g/l, NaCl = 8 g/1, Na₂HPO₄ (anhydrous) = 1.15 g/l: hereinafter referred to as PBS(-): COSMO BIO CO. Catalogue No. 320-01] containing 150 µg of the sensitized antigen, and the same amount of Freund's complete adjuvant (Iatron, Catalogue No. RM606-1) were mixed, and 0.2 ml of the resulting liquid mixture was subcutaneously administered to Lou rat (6-week old, female, CLEA JAPAN, INC.) for immunization. The same procedures were repeated as the second and third immunizations, and the immunizations using adjuvant (Hunter's TiterMax; CytRx Corporation, Ga, USA) were repeated six times.

Blood was collected from the sensitized animal, and serum was separated in an ordinary method. The antibody titer was determined by an Enzyme-antibody assay (ELISA) and Western blot technique.

### (2) Evaluation of properties of anti-HSP47 polyclonal antibody by Enzyme-antibody assay (ELISA)

The human HSP47 peptide (2-16) was dissolved in PBS(-) to obtain the peptide solution (10 µg/ml). The peptide solution was poured to wells of a rigid assay plate (Falcon; Catalogue No. 3910) at 50 µl/well, and only 50 µl of PBS(-) wad poured into the well at the edge. The plate was allowed to stand at 4 °C overnight in a wet condition. After the peptide solution was discarded, the wells were washed with PBS(-), and 100 µl of PBS(-) containing 1 % bovine serum albumin (hereinafter referred to as BSA) was poured into each well. The plate was allowed to stand at room temperature for 1 hour. After the wells were washed with PBS(-) three times, 50 µl of Lou rat serum obtained in the above (1) was added to each well. The plate was allowed to stand at room temperature for 1 hour. After the wells were washed with PBS(-) three times, 50 µl of peroxidase-labeled anti-rat IgG was poured as a second antibody to each well. The plate was allowed to stand at room temperature for 1 hour.

After the wells were washed with PBS(-) twice, 100 µl of a substrate solution prepared by dissolving one tablet (10 mg) of o-phenylene diamine (OPD) (Sigma, Catalogue No. P8287) in 10 ml of 1 M citrate buffer (pH 4.5) containing 4 µl of hydrogen peroxide solution was added dropwise to each well. The plate was allowed to stand at room temperature for 30 minutes with shielding from light. The absorption at 492 nm for each well was measured by a microplate reader (Toso, MPR-A4i type).

The sera having a high antibody titer were used as the anti-HSP47 polyclonal antibody in the following Examples.

### (3) Evaluation of properties of anti-HSP47 polyclonal antibody by western blot technique

Sodium dodecyl sulfate (SDS)-polyacrylamide gel electrophoresis of the Hela cell lysate was carried out, using Laemmli's buffer (Laemmli, N. K., "Nature", 283: pp.249-256, 1970), as follows:

A concentrated gel was prepared by mixing 6.1 ml of distilled water, 2.5 ml of 0.5 M Tris (Bio-Rad, Catalogue No. 161-0716)-HCl (pH 6.8), 100 µl of 10 % SDS (Bio-Rad, Catalogue No. 161-0301), and 1.3 ml of 30 % acrylamide (Bio-Rad, Catalogue No. 161-0101)/N,N'-methylene bisacrylamide (Bio-Rad, Catalogue No. 161-0201), deaerating for 15 minutes, and adding 50 µl of 10 % ammonium persulfate (Bio-Rad, Catalogue No. 161-0700) and 10 µl of N,N,N',N'-tetramethylethylene-diamine (hereinafter, referred to as TEMED) (Bio-Rad, Catalogue No. 161-0800).

A separating gel was prepared by slowly mixing 4.045 ml of distilled water, 2.5 ml of 1.5 M Tris-HCl (pH 8.8), 100 µl of 10 % SDS, and 3.3 ml of 30 % acrylamide/N,N'-methylene-bisacrylamide, deaerating for 15 minutes with an aspirator, and adding 50 µl of 10 % ammonium persulfate, and 5 µl of TEMED.

An electrophoresis buffer was prepared by adding 9.0 g of Tris, 43.2 g of glycine (Bio-Rad, Catalogue No. 161-0717) and 3.0 g of SDS to distilled water to obtain 600 ml of a solution, and diluting the solution with distilled water to 5 fold.

A sample buffer was prepared by mixing 2 ml of distilled water, 500 µl of 2 M Tris-HCl (pH 6.8), 0.32 g of SDS, 800 µl of β-mercaptoethanol, and 400 µl of 0.05 % (w/v) bromophenol blue (Bio-Rad, Catalogue No. 161-0404).

The HeLa cells were cultured under 5% CO₂ at 37 °C in an MEM medium containing 10 % heat inactivated fetal bovine serum (hereinafter referred to as FBS), and the lysate was prepared. SDS-polyacrylamide gel electrophoresis of the HeLa cell lysate was carried out, and the gel was brought into intimate contact with a 0.45 µm nitrocellulose membrane (Schleicher & Schuell, Catalogue No. 401196). Blotting was carried out at room temperature and 100 V for 3 hours, using a protein-transferring apparatus (Trans-Blot Electrophoretic Transfer Cell: Bio-Rad). The blotting buffer used was prepared by adding methyl alcohol to Tris-glycine buffer (pH 8.5) consisting of 0.025 M Tris and 0.192 M glycine (Tris Gly Running and Blotting Buffer; Enprotech, Mass., USA; Catalogue No. SA100034), so that the concentration of methyl alcohol became 20 %.

After blotting, the nitrocellulose membrane was dipped for 30 minutes at room temperature in PBS(-) solution containing 5 % skimmed milk (Snow Brand Milk) to perform blocking. After blocking, the first antibody reaction was carried out, using a screener blotter (Sanplatec) and the Lou rat serum obtained in the above (1) as the first antibody. The first antibody reaction was carried out in PBS(-) containing 2 % skimmed milk (Snow Brand Milk) at room temperature for 120 minutes, using 200 µl of 10-fold dilution of the Lou rat serum. After the first antibody reaction, the nitrocellulose membrane was washed, using a slow rocking shaker, by shaking twice for 5 minutes in PBS(-), four times for 15 minutes in a PBS(-) solution containing 0.1 % Tween 20 (Bio-Rad, Catalogue No. 170-6531), and further twice for 5 minutes in PBS(-).

After washing, the second antibody reaction was carried out for 2 hours, using 5 ml of a solution prepared by diluting peroxidase-labeled goat anti-rat IgG antibody (Southern Biotechnology, Catalogue No. 3030-05) 5000-fold with a PBS(-) solution containing 2 % skimmed milk. After the reaction, the nitrocellulose membrane was washed with a PBS(-) solution and a PBS(-) solution containing 0.1 % Tween 20, under the conditions same as those used in the washing after the first reaction.

After excess PBS(-) solution was removed, a western blotting detection reagent (ECL Western blotting detection reagent; Amersham, Catalogue No. RPN2106) was sprinkled over the nitrocellulose membrane, and the membrane was allowed to stand at room temperature for 1 minute. After excess detection reagent was removed, the nitrocellulose membrane was enclosed with a wrapping film. The reaction surface was contacted with an X-ray film (Kodak X-OMAT, AR; Catalogue No. 165 1454) and exposed. After developed, a band around a molecular weight of 47 kDa, i.e., the molecular weight of HSP47, was detected to evaluate the reactivity of the anti-HSP47 polyclonal antibody.

The sera whose antibody titer was observed to be high were used as the anti-HSP47 polyclonal antibody in the following Examples 3 to 8.

### Example 2: Preparation of malt extract

The malt extract used in the present Example as the substance inhibiting HSP47 production was prepared as in the method described in Example 1 in Japanese Examined Patent Publication (Kokoku) No. 7-20988. More particularly, 10 kg of barley seeds were germinated, and starch was added thereto. The whole was saccharified, filtered and dried. A part (100 g) of the saccharified residue was added to 4 liter of 0.5 N sodium hydroxide aqueous solution, extracted for 3 hours in the boiling aqueous solution, adjusting with 1 N hydrochloric acid to pH 4.0, allowed to stand for 24 hours, and centrifuged at 6000 rpm for 30 minutes, and then, the precipitates were removed. The resulting supernatant was neutralized to pH 7.0 with 1 N sodium hydroxide aqueous solution, and centrifuged at 6000 rpm for 30 minutes. The resulting supernatant was concentrated under reduced pressure, and dialyzed with running water using a dialysis cellulose tube (Sanko-Junyaku; Catalogue No. UC36-32-1000) to remove low-molecular-weight substances having molecular weights of not more than 10,000. The resulting concentrated solution was lyophilized to obtain 23 g of the dried malt extract.

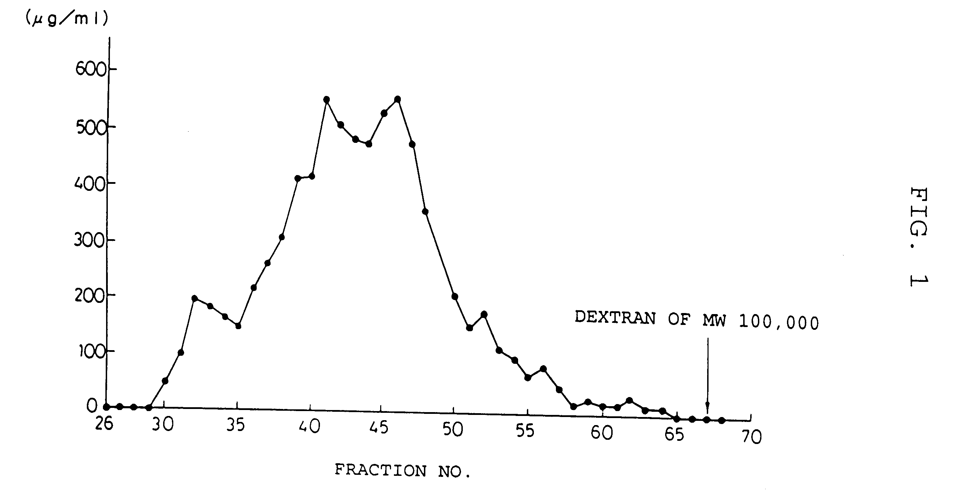

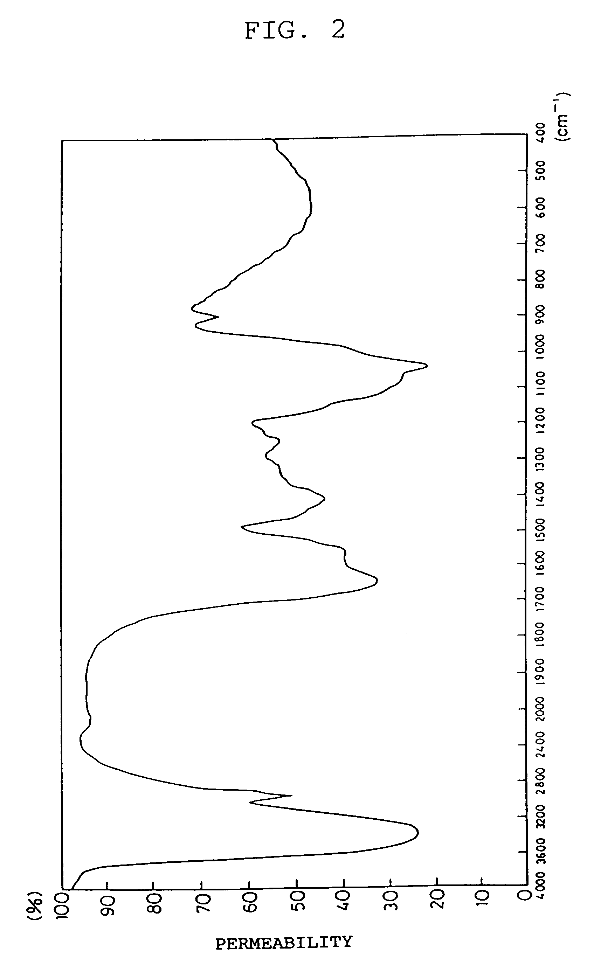

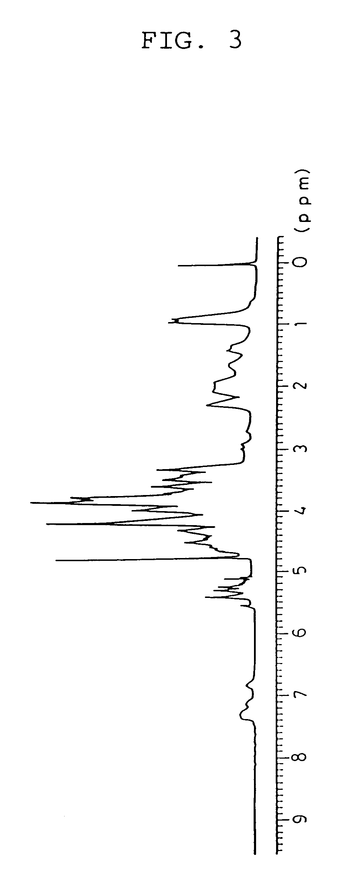

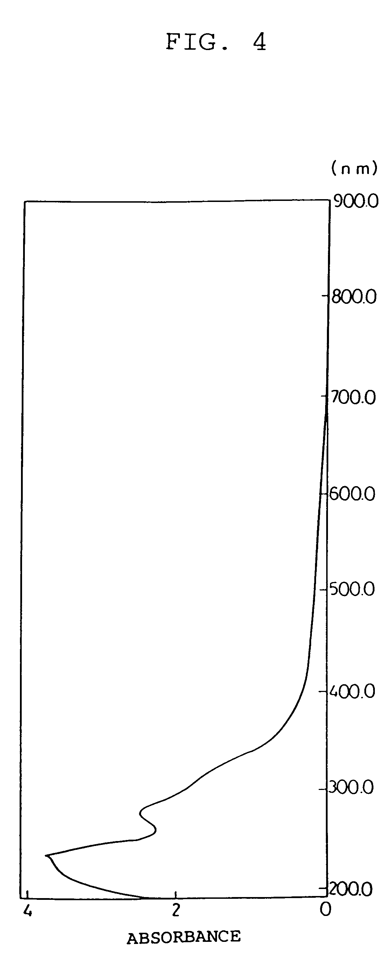

The weight-average molecular weight of the resulting dried malt extract was determined by the GPC-Lalls method to be 209,000. The resulting dried malt extract was treated with a gel chromatography [packing agent = Cellulofine GLC-2000-C column; diameter = 2.5 cm; height = 45 cm; fractionating = 3 ml/l fraction; detecting method = a phenol-sulfuric acid color reaction (490 nm); sample amount/injection = 30 mg/3 ml]. The eluting curve (chromatogram) is shown in Fig. 1. It is confirmed from Fig. 1 that the substances having molecular weights of 10,000 or less were removed. The infrared absorption spectrum was measured for the resulting dried malt extract in accordance with a KBr-tablet method by Japan Spectroscopic Co., Ltd. (JASCO) A 202 apparatus, and the result is shown in Fig. 2. Absorption peaks were observed at 3600 to 3200 cm⁻¹ and 1700 to 1600 cm⁻¹. The ¹H-nuclear magnetic resonance spectrum of the dried malt extract was measured at 500 MHz, using sodium 3-(trimethylsilyl)-1-propane sulfonate (DSS) as an internal standard and JEOL JMN-GSX500 type spectrometer, and the result is shown in Fig. 3. Peaks were observed at 0.5 to 6.0 ppm and 6.0 to 8.5 ppm. The ultraviolet-visible absorption spectrum of an aqueous solution of the dried malt extract was measured, using a multipurpose recording spectrophotometer (Shimadzu Multipurpose Recording Spectrophotometer MPS-2000), and the result is shown in Fig. 4. Absorption peaks were observed at 200 to 250 nm and 250 to 350 nm. The phenol-sulfuric acid color reaction for the dried malt extract indicated positive to show the presence of sugars. Further, the copper-Folin color reaction for the dried malt extract indicated positive to show the presence of sugars.

### Example 3: Inhibition of amount of HSP produced in human cancer cell lines by malt extract

### (1) Culture of human cancer cell lines

The following various human cancer cell lines were cultured under 5 % CO₂, at 37 °C except for the heat shock treatment period. The lung cancer cell line H69 (ATCC HTB 119) and the stomach cancer cell line KATO III (ATCC HTB 103) were cultured in an RPMI1640 medium containing 10 % inactivated FBS.

The kidney cancer cell line ACHN (ATCC CRL 1611) and the uterus cancer cell line HeLa S3 (ATCC CCL 2.2) were cultured in an MEM medium containing 10 % inactivated FBS.

The neuroblastoma cell line SK-N-MC (ATCC HTB 10) was cultured in an MEM medium containing non-essential amino acids (L-alanine = 8.9 mg/l, L-asparagine·H₂O = 15.0 mg/l, L-aspartic acid = 13.3 mg/l, L-glutamic acid = 14.7 mg/l, glycine = 7.5 mg/l, L-proline = 11.5 mg/l and L-serine = 10.5 mg/l) and 10 % inactivated FBS.

### (2) Treatment with malt extract and heat shack treatment

The malt extract prepared in Example 1 was added to each medium of the above human cancer cell lines 2 days after the inoculation. After the malt extract treatment for 24 hours, a heat shock was given at 45 °C for 15 minutes, and then, the cells were cultured at 37 °C overnight. The control tests were carried out by performing the above procedures except that the malt extract was not added.

### (3) Determination of amount of HSP produced in human cancer cell lines

The cells treated in the above (2) were homogenized, and the amounts of HSP47 produced were measured by the western blotting method.

The cells treated in the above (2) were washed with PBS(-), and 1 ml of lysis buffer [1.0 % NP-40, 0.15 M sodium chloride, 50 mM Tris-HCl (pH 8.0), 5 mM-EDTA, 2 mM-N-ethylmaleimide, 2 mM phenylmethylsulfonyl fluoride, 2 µg/ml leupeptin, and 2 µg/ml pepstatin] was added. The whole was allowed to stand for 20 minutes on ice, and then, centrifuged at 4 °C and 12000 rpm for 20 minutes. The resulting supernatant (10 µl) was added to 790 µl of PBS(-), and then, 200 µl of a protein assay dye reagent solution (Dye Reagent Concentrate: Bio-Rad Laboratories, Catalogue No. 500-0006) was added. The whole was allowed to stand at room temperature for 5 minutes, and absorbance at 595 nm was measured to determine the amounts of proteins.

For SDS-polyacrylamide gel electrophoresis (SDS-PAGE) of the samples, the protein concentration of which were measured, aliquots containing the same amount of protein were loaded onto the gel. SDS-PAGE was performed according to the method of Laemmli. After the electrophoresis, blotting and then blocking were carried out in accordance with the methods described in Example 1. More particularly, the gel was brought into contact with a 0.45 µm nitrocellulose membrane (Schleicher & Schuell, Catalogue No. 401196) at room temperature and 100 V, using a protein-transferring apparatus (Trans-Blot Electrophoretic Transfer Cell: Bio-Rad), and blotting was carried out for 3 hours. The blotting buffer used was same as that used in Example 1 (3). After blotting, the nitrocellulose membrane was incubated in a 10 % skimmed milk-PBS(-) solution at room temperature for 30 minutes to block non-specific bindings.

After blocking, the first antibody reaction was carried out on the nitrocellulose membrane, using a screener blotter (Sanplatec), and the anti-human-HSP47 rat polyclonal antibody prepared in Example 1. Thereafter, the nitrocellulose membrane was washed with two fresh PBS(-)'s for 5 minutes, using a slow rocking shaker, then, with four fresh PBS(-)-0.1 % Tween 20 (Bio-Rad, Catalogue No. 170-6531) solutions for 15 minutes, and finally, with two fresh PBS(-)'s for 5 minutes.

After washing, the second antibody reaction was carried out for 2 hours, using 5 ml of a solution prepared by diluting peroxidase-labeled goat anti-rat IgG antibody (Southern Biotechnology, Catalogue No. 3030-05) 5000-fold with a PBS(-) solution containing 2 % skimmed milk. After the reaction, the nitrocellulose membrane was washed with two fresh PBS(-) solutions for 5 minutes, and with five fresh PBS(-)-0.1 % Tween 20 solutions for 15 minutes, using a slow rocking shaker, and finally, with two fresh PBS(-)'s for 5 minutes. After excess PBS(-) solution was removed, a western blotting detection reagent (ECL Western blotting detection reagent; Amersham, Catalogue No. RPN2106) was sprinkled over the nitrocellulose membrane, and the membrane was incubated for 1 minute. After excess detection reagent was removed, the nitrocellulose membrane was enclosed with a wrapping film. The reaction surface was contacted with an X-ray film (Kodak X-OMAT, AR; Catalogue No. 165 1454) and exposed. After developed, the presence of HSP47 was detected. The results are shown in Table 1. In Table 1, the symbol 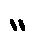 ↓*"* means that the amounts of HSP47 produced were reduced by the malt extract-treatment in comparison with the control tests.

**Table 1**

| cancer | cancer cell line | change of amount of HSP47 produced |
|---|---|---|
| lung | H69 | ↓ |
| uterus | HeLa S 3 | ↓ |
| stomach | KATO III | ↓ |
| kidney | ACHN | ↓ |
| nerve | SK-N-MC | ↓ |

It is apparent from Table 1 that the malt extract inhibits the HSP47 production in the lung cancer cell line H69, the uterus cancer cell line HeLa S3, the stomach cancer cell line KATO III, the kidney cancer cell line ACHN, and the neuroblastoma cell line SK-N-MC. Therefore, it can be concluded that the malt extract has an activity to inhibit the HSP47 production, and is useful as the substance inhibiting HSP47 production. Further, the above results show that the malt extract inhibits overproduction of the extracellular matrix.

### Example 4: Inhibition of amount of HSP produced in human cancer cell lines by flavonoid derivatives

The procedure described in Example 3 was performed, except that one of the following flavonoid derivatives was used instead of the malt extract, and the lung cancer cell line H69, the colon cancer cell line COLO 205 (ATCC CCL 222), the kidney cancer cell line ACHN, the uterus cancer cell line HeLa S3, and the neuroblastoma cell line SK-N-MC were used. The flavonoid derivatives used and the concentrations in the medium were 100 µM quercetin (Nacalai tesque), 100 µM rutin, 100 µM catechin [(+) -Catechin; Funakoshi; Code No. 0952: EXTRASYN THESE, France], and 10 µM baicalein. The colon cancer cell line COLO 205 was cultured in an RPMI1640 medium containing 10 % inactivated FBS. The results are shown in Table 2. In Table 2, the symbol 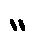 ↓*"* means that the amounts of HSP47 produced were reduced by the flavonoid-treatment in comparison with the control tests.

**Table 2**

| cancer | cancer cell line | quercetin | rutin | catechin | baicalein |
|---|---|---|---|---|---|
| lung | H69 | | | ↓ | |
| uterus | HeLa S 3 | ↓ | | ↓ | ↓ |
| colon | COLO 205 | ↓ | | ↓ | |
| kidney | ACHN | ↓ | | | |
| nerve | SK-N-MC | | ↓ | ↓ | |

It is apparent from Table 2 that catechin inhibits the HSP47 production in the lung cancer cell line H69, the uterus cancer cell line HeLa S3, the colon cancer cell line COLO 205, and the neuroblastoma cell line SK-N-MC; that quercetin inhibits the HSP47 production in the uterus cancer cell line HeLa S3, the colon cancer cell line COLO 205, and the kidney cancer cell line ACHN; that rutin inhibits the HSP47 production in the neuroblastoma cell line SK-N-MC; and that baicalein inhibits the HSP47 production in the uterus cancer cell line HeLa S3. Therefore, it can be concluded that catechin, quercetin, rutin and baicalein have an activity to inhibit the HSP47 production as the substance inhibiting HSP47 production. Further, the above results show that the flavonoids, such as catechin, quercetin, rutin and baicalein, inhibit overproduction of the extracellular matrix.

### Example 5: Inhibition of amount of HSP produced in human cancer cell lines by PSK

The procedure described in Example 3 was performed, except that PSK (the final concentration = 1 mg/ml: the trade name = Krestin; Sankyo Co., Ltd.) was used instead of the malt extract, and the lung cancer cell line H69, the stomach cancer cell line KATO III, the prostate cancer cell line DU145 (ATCC HTB 81), the colon cancer cell line COLO 205, the kidney cancer cell line ACHN, the uterus cancer cell line HeLa S3, the prostate cancer cell line PC-3 (ATCC CRL 1435), and the neuroblastoma cell line SK-N-MC were used.

The prostate cancer cell line DU145 and the colon cancer cell line COLO 205 were cultured in an RPMI1640 medium containing 10 % inactivated FBS (Sigma, Catalogue No. 3520).

The prostate cancer cell line PC-3 was cultured in an F-12K medium containing 7 % inactivated FBS (Sigma, Catalogue No. 3520).

The results are shown in Table 3. In Table 3, the symbol 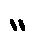 ↓*"* means that the amounts of HSP47 produced were reduced by the PSK-treatment in comparison with the control tests.

**Table 3**

| cancer | cancer cell line | change of amount of HSP47 produced |
|---|---|---|
| lung | H69 | ↓ |
| uterus | HeLa S 3 | ↓ |
| stomach | KATO III | ↓ |
| kidney | ACHN | ↓ |
| prostate | DU 145 | ↓ |
| prostate | PC-3 | ↓ |
| nerve | SK-N-MC | ↓ |

It is apparent from Table 3 that PSK inhibits the HSP47 production in the lung cancer cell line H69, the uterus cancer cell line HeLa S3, the colon cancer cell line COLO 205, the stomach cancer cell line KATO III, the kidney cancer cell line ACHN, the prostate cancer cell line DU145, the prostate cancer cell line PC-3, and the neuroblastoma cell line SK-N-MC. Therefore, it can be concluded that PSK has an activity to inhibit the HSP47 production, and is useful as the substance inhibiting HSP47 production. Further, the above results show that PSK inhibits overproduction of the extracellular matrix.

### Example 6: Inhibition of amount of HSP produced in human cancer cell lines by paeoniflorin

The procedure described in Example 3 was performed, except that paeoniflorin (the final concentration = 100 mM: manufactured by Matsuura Yakugyo, available from Kishida Kagaku) was used instead of the malt extract, and the uterus cancer cell line HeLa S3 was used as the human cancer cell line.

The result showed that paeoniflorin inhibits the HSP47 production in the uterus cancer cell line HeLa S3. Therefore, it can be concluded that paeoniflorin has an activity to inhibit the HSP47 production as the substance inhibiting HSP47 production. Further, the above results show that paeoniflorin inhibits overproduction of the extracellular matrix.

### Example 7: Inhibition of amount of HSP produced in human cancer cell lines by α-tocopherol

The procedure described in Example 3 was performed, except that D-α-tocopherol (the final concentration = 20 µM: Funakoshi; Catalogue No. ICN 81-1521-47) was used instead of the malt extract, and the uterus cancer cell line HeLa S3 was used as the human cancer cell line.

The result showed that α-tocopherol inhibits the HSP47 production in the uterus cancer cell line HeLa S3. Therefore, it can be concluded that α-tocopherol has an activity to inhibit the HSP47 production as the substance inhibiting HSP47 production. Further, the above results show that α-tocopherol inhibits overproduction of the extracellular matrix.

### Example 8: Inhibition of amount of HSP produced in human cancer cell lines by ferulic acid

The procedure described in Example 3 was performed, except that ferulic acid (the final concentration = 100 µM: Kishida Kagaku; Catalogue No. 268-38963) was used instead of the malt extract, and the uterus cancer cell line HeLa S3 was used as the human cancer cell line.

The result showed that ferulic acid inhibits the HSP47 production in the uterus cancer cell line HeLa S3. Therefore, it can be concluded that ferulic acid has an activity to inhibit the HSP47 production as the substance inhibiting HSP47 production. Further, the above results show that ferulic acid inhibits overproduction of the extracellular matrix.

As explained above, the pharmaceutical composition of the present invention contains the substance inhibiting HSP47 production, and therefore, has an activity to improve collagen overproduction observed in cells suffering from the diseases exhibiting pathosis of overproduction of the extracellular matrix, for example, liver cirrhosis, interstitial lung disease, chronic renal failure (or disease leading thereto), postoperative scar, burn scar, keloid or hypertrophic scar remaining after a traffic accident, scleroderma, arteriosclerosis, or rheumatoid arthritis. Accordingly, the administration of the pharmaceutical composition of the present invention can inhibit the fibrosis and the sclerosis of organs or tissues, efficiently improve physiological conditions of a patient suffering from such diseases, and efficiently treat such diseases.

Further, the pharmaceutical composition of the present invention is useful for preventing or treating various diseases accompanied with abnormal growth of the vascularization.

Furthermore, it is known that the interstitial stroma having a basic structure of the type I collagen and fibronectin serves, in cancer metastasis, as a guide for the released cancer-cells to enter the vessels in the vicinity. Therefore, the cancer metastasis may be inhibited by administering the pharmaceutical composition of the present invention.

Although the present invention has been described with reference to specific embodiments, various changes and modifications obvious to those skilled in the art are deemed to be within the spirit, scope, and concept of the invention.

## Claims

1. A pharmaceutical composition comprising a substance inhibiting HSP47 production, selected from the group consisting of a malt extract, a flavonoid compound, a protein-bound-polysaccharide obtained from a fungus belonging to Coriolus versicolor, a paeoniflorin derivative, a tocopherol derivative, and a ferulic acid derivative, and a pharmaceutically acceptable carrier.

2. The composition according to claim 1, wherein the malt extract is a product prepared by saccharifying a malt, filtering a resulting saccharified malt, extracting a resulting filter cake from the saccharified malt with water, a hydrophilic solvent or a mixture thereof, and removing low molecular weight fractions from a resulting liquid extract.

3. The composition according to claim 2, wherein the extraction of the filter cake from the saccharified malt with water, the hydrophilic solvent or the mixture thereof is carried out in an alkaline condition, a resulting liquid extract is neutralized, a resulting precipitated substance is removed, and a low molecular weight fraction of a molecular weight of not more than 10,000 is removed from the resulting supernatant.

4. The composition according to claim 2, wherein a liquid extract obtained by the extraction of the filter cake from the saccharified malt with water, the hydrophilic solvent or the mixture thereof is acidified to pH 3 to 5, a precipitated protein is removed, a resulting supernatant is neutralized, a resulting precipitated substance is removed, and a low molecular weight fraction of a molecular weight of not more than 10,000 is removed from a resulting supernatant.

5. The composition according to claim 2 or 4, wherein the extraction of the filter cake from the saccharified malt with water, the hydrophilic solvent or the mixture thereof is carried out in an alkaline condition.

6. The pharmaceutical composition according to claim 1, wherein the flavonoid compound is quercetin, rutin, baicalein, or catechin.

7. The pharmaceutical composition according to claim 6, wherein the catechin is at least one compound selected from the group consisting of epigallocatechin gallate, epicatechin gallate, epigallocatechin, epicatechin, and isomers thereof.

8. The pharmaceutical composition according to claim 1, wherein the protein-bound-polysaccharides is obtained from mycelium, broth, or fruit body of a fungus belonging to Coriolus versicolor.

9. The composition according to claim 8, wherein the protein-bound-polysaccharides is PSK.

10. The pharmaceutical composition according to claim 1, wherein the paeoniflorin derivative is paeoniflorin.

11. The pharmaceutical composition according to claim 1, wherein the tocopherol derivative is α-tocopherol.

12. The pharmaceutical composition according to claim 1, wherein the ferulic acid derivative is ferulic acid or a pharmaceutically acceptable salt thereof.

13. The pharmaceutical composition according to claim 1, comprising a tea extract containing a tea catechin as the substance inhibiting HSP47 production.

14. The pharmaceutical composition according to claim 1, comprising an extract from a plant containing paeoniflorin, as the substance inhibiting HSP47 production.

15. The pharmaceutical composition according to claim 1, comprising an extract from a peony root, as the substance inhibiting HSP47 production.

16. The pharmaceutical composition according to claim 1, comprising an extract from a plant containing ferulic acid, as the substance inhibiting HSP47 production.

17. The pharmaceutical composition according to claim 1, comprising an extract from a rice, as the substance inhibiting HSP47 production.

18. Use of a substance inhibiting HSP47 production, selected from the group consisting of a malt extract, a flavonoid compound, a protein-bound-polysaccharides from a fungus belonging to Coriolus versicolor, a paeoniflorin derivative, a tocopherol derivative, and a ferulic acid derivative, for preparing a pharmaceutical composition.
